# EUROPÄISCHE PATENTSCHRIFT

(11) **EP 3 117 840 B1**
(45) Veröffentlichungstag und Bekanntmachung des Hinweises auf die Patenterteilung: **29.08.2018**
(21) Anmeldenummer: 16001505.3
(22) Anmeldetag: 06.07.2016
(51) Int. Cl.: A61L 2/26, F16K 11/052, F16K 15/03, F16K 1/22, A61L 2/07

(54) **BEHANDLUNGS- UND TRANSPORTBEHÄLTER MIT EINEM KLAPPENVENTIL SOWIE VERFAHREN ZUM ENTLEEREN EINES SOLCHEN**
TREATMENT AND TRANSPORT CONTAINER WITH A BUTTERFLY VALVE AND METHOD FOR EMPTYING IT
RECIPIENT DE TRANSPORT ET DE TRAITEMENT COMPRENANT UNE SOUPAPE A CLAPET ET PROCEDE DE VIDANGE D'UN TEL RECIPIENT

(30) Priorität: 17.07.2015 DE 102015009083
(43) Veröffentlichungstag der Anmeldung: 18.01.2017
(73) Patentinhaber: Atec Pharmatechnik Gmbh, 24966 Sörup (DE)
(72) Erfinder: Mumm, Hans-Werner, 24966 Sörup (DE)
(74) Vertreter: Thomas, Götz

(56) Entgegenhaltungen:
- EP-A1- 2 502 636
- EP-A2- 1 835 238
- WO-A1-2008/058454
- WO-A1-2010/040383
- WO-A2-95/27163
- WO-A2-2011/002879
- CN-A- 1 236 871
- US-A1- 2013 340 862

## Beschreibung

Die Erfindung betrifft einen Behandlungs- oder Transportbehälter gemäß dem Oberbegriff des Anspruchs 1 zur Behandlung und/oder zum Transport von kleineren Gegenständen, wie Verschlusskappen aus Aluminium. Die Erfindung betrifft weiter ein Verfahren zum Entleeren eines solchen Behandlungs- oder Transportbehälters gemäß dem Oberbegriff des Anspruchs 11.

Behandlungs- oder Transportbehälter der eingangs genannten Art mit einem erweiterten Körperteil und einem verengten Halsteil, der eine Befüll- und Entleer-Öffnung umgibt, sowie mit einem im Halsteil angeordneten Klappenventil sind beispielsweise aus der EP 1 449 543 B1 oder der EP 1.510 227 B1 der Anmelderin bekannt. Unter den Gegenständen, die in diesen Behältern behandelt und/oder transportiert werden, befinden sich auch Verschlusskappen aus Aluminium, die zum Beispiel zum Verschließen von kleinen Arzneimittelbehältern, wie Vials, dienen. Diese Verschlusskappen sind verhältnismäßig dünnwandig, so dass sie sich leicht irreversibel verformen, wenn während der Behandlung oder des Transports übermäßige Kräfte auf die Verschlusskappen ausgeübt werden. Verformte Verschlusskappen wiederum können in einem nachfolgenden Abfüllprozess zu Betriebsstörungen führen, da sie sich nicht auf den Vials anbringen lassen. Daher muss dafür Sorge getragen werden, dass es nicht zu derartigen Verformungen oder Beschädigungen des im Behälter behandelten oder transportierten Gutes kommt.

Eine von der Anmelderin ermittelte Ursache für Verformungen von Aluminium-Verschlusskappen besteht darin, dass die Behandlungs- oder Transportbehälter der eingangs genannten Art mit einem Klappen- oder Schmetterlingsventil im Halsteil ausgestattet sind, das eine einzige Ventilklappe aufweist. Wie bei Klappen- oder Schmetterlingsventilen üblich, weist diese Ventilklappe einen dem Öffnungsquerschnitt der Befüll- und Entleer-Öffnung entsprechenden Umriss auf und ist mittels eines Drehantriebs um eine diametral durch die Befüll- und/oder Entleer-Öffnung verlaufende Schwenkachse zwischen zwei Endstellungen verschwenkbar, wobei sie in der einen Endstellung senkrecht zur Längsmittelachse des Halsteils ausgerichtet ist und die Befüll- und Entleer-Öffnung versperrt, während sie in der anderen Endstellung parallel zur Längsmittelachse des Halsteils ausgerichtet ist und die Befüll- und Entleer-Öffnung freigibt. Wenn das Klappen- oder Schmetterlingsventil zum Entleeren des Behälters geöffnet werden soll, während die Befüll- und Entleer-Öffnung nach unten weist, liegen die im Behälter behandelten oder transportierten Verschlusskappen vor dem Öffnen als Schüttung auf der nach oben weisenden Seite der Ventilklappe. Wenn das Ventil dann geöffnet wird, schwingt eine Hälfte der Ventilklappe nach unten, während ihre andere Hälfte nach oben in die Schüttung schwingt.

Dabei werden erstens die oberhalb von dieser Hälfte liegenden Verschlusskappen entgegen dem Gewicht der Schüttung angehoben, was ausreichen kann, um Verformungen einzelner Verschlusskappen zu verursachen, und zwar vor allem dann, wenn die Ventilklappe relativ schnell in die Offenstellung verschwenkt wird, um die Entleerung des Behälters zu beschleunigen.

Bei anderen, schwereren Gütern, wie Verschlusskappen aus Gummi, wird vor allem zu Beginn der Schwenkbewegung der Ventilklappe in die Offenstellung die Schüttung der Gegenstände oberhalb von der nach oben schwingenden Hälfte der Ventilklappe etwas zusammengedrückt oder komprimiert, was dort anschließend zu einer Brückenbildung führen kann, welche die Entleerung des Behälters erschwert oder zumindest verlangsamt.

Drittens werden vor allem während des mittleren Teils der Schwenkbewegung der Ventilklappe in die Offenstellung alle kleineren Gegenstände, die als Schüttung auf der schräg geneigten Oberseite der Ventilklappe liegen, von dieser zu derjenigen Seite der Schwenkachse hin gelenkt, auf der sich die Ventilklappe nach unten bzw. vom Körperteil des Behälters weg bewegt. Dies hat zur Folge, dass diese Gegenstände auf der Oberseite der Ventilklappe in einer Richtung nach unten rutschen, wodurch sich auf dieser Seite der Schwenkachse mehr Gegenstände in Richtung des sich öffnenden Spaltes zwischen dem Rand der Ventilklappe und der benachbarten Begrenzungswand des Halsteils bewegen. Auf der anderen Seite der Schwenkachse bewegen sich hingegen nur diejenigen Gegenstände durch den sich öffnenden Spalt zwischen dem Rand der Ventilklappe und der benachbarten Begrenzungswand des Halsteils, die sich unmittelbar oberhalb von diesem Spalt befinden. Obwohl die sich öffnenden Spalte auf beiden Seiten der Schwenkachse stets dieselbe Größe aufweisen, fällt dadurch eine ungleiche Anzahl von Gegenständen durch die beiden Spalte, was die Entleerung des Behälters ebenfalls verlangsamt.

Weitere Behälter der eingangs genannten Art sind zum Beispiel in der DE 44 09 659 A1 oder der WO 00/61199 A1 offenbart, wo die oben genannten Probleme in ähnlicher Weise auftreten können. Auch die WO 2010/040383 A1 und die EP 2 502 636 A1 offenbaren ähnliche Behälter.

Aus der DE 42 09 508 A1 ist bereits ein Silobehälter für schwer rieselbares Gut mit den Merkmalen im Oberbegriff des Anspruchs 1 bekannt. Bei dem Klappenventil dieses Silobehälters sind die halbkreisförmigen Ventilklappen um parallele Schwenkachsen schwenkbar, die im Abstand voneinander angeordnet sind. Darüber ist eine Abdeckung in Form eines Winkelblechs vorgesehen, die verhindert, dass oberhalb von den Schwenkachsen Gut im Behälter zurückgehalten wird, wenn beide Klappen geöffnet sind. Jedoch verhindert eine solche Abdeckung, dass die Entleer-Öffnung auch als Befüll-Öffnung verwendet werden kann. Darüber hinaus hält die Abdeckung Gut im Behälter zurück, wenn nur eine der Klappen geöffnet wird. Der Abstand der Schwenkachsen bewirkt zudem eine gewisse Reduzierung des Öffnungsquerschnitts.

Die EP 1 835 238 A2, die WO 95/27163 A2, die US 2013/340862 A1, die WO 2008/058454 A1, die WO 2011/002879 A2 und die CN 1 236 871 A1 offenbaren in Leitungen angeordnete Rückschlagventile, die jeweils zwei separate, zwischen einer Offenstellung und einer Schließstellung verschwenkbare Ventilklappen aufweisen, welche zum Teil fluchtende Schwenkachsen aufweisen.

Ausgehend hiervon liegt der Erfindung die Aufgabe zugrunde, einen Behandlungs- oder Transportbehälter sowie ein Verfahren der eingangs genannten Art dahingehend zu verbessern, dass bei der Entleerung des Behälters ein Zurückhalten von Gut im Behälter und eine Beschädigung von empfindlichem Gut sicher verhindert werden und dass das Klappenventil ohne Modifikation auch für Behälter verwendet werden kann, die nur eine einzige Befüll- und Entleer-Öffnung aufweisen.

Bei dem Behandlungs- oder Transportbehälter wird diese Aufgabe erfindungsgemäß durch die Merkmalskombination des Anspruchs 1 gelöst.

Durch die erfindungsgemäße Merkmalskombination kann das Klappenventil ohne die Notwendigkeit einer Modifikation auch für Behälter verwendet werden, die nur eine einzige Befüll- und Entleer-Öffnung besitzen, sofern bei diesen Behältern gemäß einer bevorzugten Ausgestaltung der Erfindung mindestens eine der Ventilklappen und vorzugsweise beide Ventilklappen um insgesamt etwa 180 Grad schwenkbar sind. Bei gegebenem Innenquerschnitt des Halsteils des Behälters wird zudem der Öffnungsquerschnitt der Befüll- und Entleer-Öffnung in der Offenstellung der Ventilklappen maximiert und ein Zurückhalten von Gut im Behälter verhindert, weil dann die Projektionsfläche der beiden Ventilklappen in einer zur Längsmittelachse des Halsteils senkrechten Ebene minimiert und weniger Platz als bei zwei nebeneinander liegenden parallelen Schwenkachsen benötigt wird. Darüber hinaus können in der Offenstellung gegenüberliegende ebene Breitseiten der beiden Ventilklappen in paralleler Ausrichtung in flächige Anlage gebracht werden.

Durch die Ausbildung des Klappenventils mit zwei beweglichen Ventilklappen lässt sich beim Öffnen des Klappenventils in der Entleer-Stellung ein Anheben von Gegenständen in der Schüttung und damit eine Verformung von druckempfindlichen Gegenständen vermeiden. Dadurch, dass die Ventilklappen einzeln oder gemeinsam vom Körperteil weg nach unten verschwenkt werden, kommt es stattdessen bereits zu Beginn der Schwenkbewegung zu einer Auflockerung der gesamten Schüttung oberhalb des Klappenventils.

Um das Befüllen des Behälters bei nach oben weisendem Halsteil zu erleichtern, sind die beiden Ventilklappen vorteilhaft auch beide in Richtung des Körperteils verschwenkbar, um in einer Befüll-Stellung des Behälters die Befüll- und Entleer-Öffnung mindestens teilweise freizugeben. In Verbindung mit der erfindungsgemäßen Ausgestaltung des Behälters bedeutet dies, dass beide Ventilklappen vorteilhaft um einen Winkel von 180 Grad oder von nahezu 180 Grad zwischen zwei Endstellungen verschwenkbar sind, in denen sie bei geöffnetem Klappenventil parallel oder nahezu parallel zueinander ausgerichtet sind und jeweils eine Hälfte des Öffnungsquerschnitts der Befüll- und Entleer-Öffnung freigeben, während sie in ihrer Mittenstellung bei geschlossenem Klappenventil eine Ebene aufspannen und die Befüll- und Entleer-Öffnung versperren.
Jedoch kann es auch ausreichen, wenn die beiden Ventilklappen in der Entleer-Stellung aus ihrer Schließstellung nur jeweils um einen Winkel von 90 Grad vom Körper weg nach unten schwenkbar sind.

Um vor der Entleerung des Behälters in dessen umgedrehter Entleer-Stellung etwaige im Behälter verbliebene restliche flüssige Behandlungsmedien schnell aus dem Behälter austreten zu lassen, die behandelten Gegenstände jedoch zurückzuhalten, sind die beiden Ventilklappen vorteilhaft mit einer Mehrzahl von Löchern versehen, so dass die Behandlungsmedien die Ventilklappen nicht nur entlang ihres Umfangsrandes passieren können, sondern auch durch die Löcher der Ventilklappen hindurchtreten können.

Dort, wo die Befüll- und Entleer-Öffnung des Behälters zum Andocken an einen Rapid-Transfer-Port bestimmt ist, wie bei dem Behälter aus der EP 1 510 227 B1, gewährleistet die Ausbildung der Ventilklappen mit Löchern, dass die Behandlungsmedien bei der Behandlung und insbesondere bei der Sterilisation bis zum Rapid-Transfer-Port gelangen, dass jedoch die zu behandelnden kleinen Gegenstände nicht bis dorthin mitgeführt werden können.

Um es zu ermöglichen, die beiden Ventilklappen unabhängig voneinander zu verschwenken, ist der Behälter bevorzugt mit zwei getrennten, einzeln steuerbaren Drehantrieben versehen, von denen jeder zum Verschwenken von einer der Ventilklappen dient. Die Drehantriebe sind zweckmäßig an der Außenseite des Halsteils angeordnet und weisen jeweils eine Antriebswelle auf, die sich in radialer Richtung durch eine Wandöffnung einer Begrenzungswand des Halsteils hindurch erstreckt und drehfest mit der zugehörigen Ventilklappe verbunden ist, vorzugsweise durch Schweißen. Aufgrund der Fluchtung der Schwenkachsen der beiden Ventilklappen liegen sich die Wandöffnungen für die beiden Antriebswellen im Halsteil diametral gegenüber.

Um einen Austritt von Behandlungsmedien entlang der beiden Antriebswellen zu verhindern, sind die beiden Wandöffnungen abgedichtet, vorteilhaft mittels eines in den Halsteil des Behälters eingesetzten Dichtungsrings, der mit zwei radialen Durchgangsöffnungen für die beiden Antriebswellen versehen ist.

Vorteilhaft besitzt der Halsteil des Behälters einen runden Öffnungsquerschnitt, während jede der beiden Ventilklappen einen halbkreisförmigen Umriss aufweist, der von einem geraden Rand und einem kreisbogenförmigen Rand begrenzt wird. Jede der Antriebswellen ist bevorzugt einstückig mit der zugehörigen Ventilklappe verbunden, wobei sie sich in radialer Richtung der Befüll- und Entleer-Öffnung entlang von einer Hälfte des geraden Randes erstreckt.

Um sicherzustellen, dass beim auch beim Schwenken des Behälters die erfindungsgemäße Fluchtung der Schwenkachsen der beiden Ventilklappen trotz einer auf den Ventilklappen lastenden Schüttung der Gegenstände erhalten bleibt, ist eine der beiden Antriebswellen an ihrem radial inneren Ende mit einem zylindrischen oder konischen Zentrier- oder Führungszapfen versehen, während die andere der beiden Antriebswellen an ihrem radial inneren Ende mit einer komplementären zylindrischen oder konischen Zentrier- oder Führungsbohrung versehen ist. Durch die rotationssymmetrische Ausbildung des Zapfens und der Bohrung werden die inneren Enden der beiden Antriebswellen drehbar miteinander verbunden, so dass die beiden Ventilklappen mit unterschiedlichen Drehrichtungen und unabhängig von einander verschwenkbar sind. Vorteilhaft erstreckt sich dabei die mit der Bohrung versehen Antriebswelle bis zur Längsmittelachse des Halsteils, während sich die andere Antriebswelle mit dem Zapfen über die Längsmittelachse des Halsteils hinaus erstreckt.

Um die Montage des Klappenventils zu erleichtern, weist vorteilhaft jede Antriebswelle zwei Abschnitte auf, von denen einer über den Drehantrieb übersteht und der andere starr mit der Ventilklappe verbunden ist. Die beiden Wellenabschnitte jeder Antriebswelle werden bei der Montage des Drehantriebs drehfest miteinander verbunden, zum Beispiel indem ein Mehrkantzapfen am freien Ende eines Wellenabschnitts in eine Mehrkantbuchse am gegenüberliegenden freien Ende des anderen Wellenabschnitts gesteckt wird. Dies gestattet es, zuerst die beiden an den Ventilklappen angeschweißten Wellenabschnitte drehbar miteinander zu verbinden und dann die starr mit diesen verbundenen, radial nach außen weisenden Wellenabschnitte unter Verformung des Dichtungsrings von innen her durch die beiden Durchgangsöffnungen im Dichtungsring hindurchzuführen, bevor zuletzt die beiden Drehantriebe an der Außenseite des Halsteils montiert und dabei die über die Drehantriebe überstehenden Wellenabschnitte drehfest mit den durch die Durchgangsöffnungen geführten Wellenabschnitten verbunden werden.

Bei dem Verfahren zum Entleeren des erfindungsgemäßen Behälters wird der Behälter in seine Entleer-Stellung geschwenkt, in welcher der Halsteil vertikal oder schräg nach unten weist, und dann in der Entleer-Stellung das Klappenventil geöffnet. Dabei wird erfindungsgemäß mindestens eine der beiden Ventilklappen unter Freigabe von mindestens einem Teil der Befüll- und Entleer-Öffnung um zwei fluchtende Schwenkachsen der beiden Ventilklappen vom Körperteil weg nach unten geschwenkt. Vorzugsweise werden beide Ventilklappen zugleich vom Körperteil weg nach unten geschwenkt, um die Befüll- und Entleer-Öffnung schneller freizugeben und dadurch die Entleerung des Behälters zu beschleunigen.

Vorteilhaft werden die beiden Ventilklappen beim Öffnen des Klappenventils in der Entleer-Stellung des Behälters synchron, das heißt mit gleicher Geschwindigkeit, nach unten verschwenkt, so dass der sich öffnende Spalt beiderseits der Schwenkachse stets etwa dieselbe Größe besitzt, was in Verbindung mit den nach unten zum Spalt hin geneigten Oberflächen der beiden Ventilklappen für eine schnellere Entleerung des Behälters und einen gleichmäßigeren Austritt der Gegenstände beiderseits von der Schwenkachse der Ventilklappen sorgt.

Dort, wo die Befüll- und Entleer-Öffnung des Behälters zum Andocken an einen Rapid-Transfer-Port bestimmt ist, wie bei dem Behälter aus der EP 1 510 227 B1, erfolgt die Schwenkbewegung des Behälters vorzugsweise in einer Schwenkebene, die senkrecht zu der Schwenkachse der beiden Ventilklappen ausgerichtet ist. Anders als die Schwenkachse der einteiligen Ventilklappe des Klappenventils des Behälters aus der EP 1 510 227 B1 ist daher die Schwenkachse der beiden Ventilklappen hier nicht parallel sondern senkrecht zur Schwenkebene des Behälters ausgerichtet, wodurch die Behandlungsmedien gleichmäßiger durch den gesamten Rapid-Transfer-Port hindurchgeleitet werden.

Im Folgenden wird die Erfindung anhand eines in der Zeichnung dargestellten Ausführungsbeispiels näher erläutert.
Fig. 1 zeigt eine Seitenansicht eines erfindungsgemäßen Behandlungs- und Transportbehälters in aufrechter Stellung;
Fig. 2 zeigt eine Seitenansicht eines verjüngten Oberteils des Behälters von der entgegengesetzten Seite her;
Fig. 3 zeigt eine perspektivische Ansicht eines Halsteils des Behälters mit zwei Drehantrieben eines im Halsteil angeordneten Klappenventils bei aufrechtem Behälter;
Fig. 4 zeigt eine perspektivische Ansicht des Klappenventils und der Drehantriebe entsprechend Fig. 3 bei geschlossenem Klappenventil;
Fig. 5 zeigt eine Ansicht entsprechend Fig. 4, jedoch bei geöffnetem Klappenventil;
Fig. 6 zeigt eine Ansicht entsprechend Fig. 4 und 5, jedoch bei teilweise geöffnetem Klappenventil;
die Figuren 7 bis 9 zeigen andere perspektivische Ansichten des Klappenventils und der Drehantriebe in den Stellungen der Fig. 4, Fig. 5 bzw. Fig. 6 bei aufrechtem Behälter;
Fig. 10 zeigt eine auseinandergezogene perspektivische Ansicht des Behälteroberteils und des Klappenventils;
Fig. 11 zeigt eine Oberseitenansicht des Behälteroberteils mit dem Klappenventil;
Fig. 12 zeigt eine teilweise geschnittene Seitenansicht des des Behälteroberteils mit dem Klappenventil entlang der Linie A-A der Fig. 11;
Fig. 13 zeigt eine Schnittansicht des Behälteroberteils mit dem Klappenventil entlang der Linie B-B der Fig. 11;
Fig. 14 zeigt eine vergrößerte Ansicht des Ausschnitts C in Fig. 12;
Fig. 15 zeigt eine vergrößerte Ansicht des Ausschnitts D in Fig. 13.

Der in der Zeichnung dargestellte erfindungsgemäße Behandlungs- und Transportbehälter 10 dient unter anderem zur Reinigung und Sterilisation von Verschlusskappen aus Aluminium für Vials oder für andere pharmazeutische Verpackungsbehälter, sowie zum Transport der Verschlusskappen vor und nach der Reinigung und Sterilisation. Jedoch kann der Behälter 10 auch zur Reinigung und Sterilisation von anderen kleineren Gegenständen oder nur zum Transport der gereinigten Gegenstände eingesetzt werden. Der Behälter 10 wird vor allem in der pharmazeutischen Industrie eingesetzt.

Wegen der hohen Anforderungen an die Reinheit erfolgt die Behandlung der Verschlusskappen ebenso wie das Befüllen und das Entleeren des Behälters 10 innerhalb eines Reinraums, bevorzugt mit Hilfe einer Reinigungs- und Sterilisationsvorrichtung, wie sie zum Beispiel in der EP 1 449 543 B1 oder der EP 1 510 227 B1 der Anmelderin beschrieben ist. Dabei kann das Befüllen des Behälters 10 mit den unbehandelten Verschlusskappen und das Entleeren des Behälters 10 direkt an dieser Vorrichtung erfolgen, oder alternativ an einer Befüll- bzw. Übergabe-Station, zu welcher der Behälter 10 nach der Abnahme von der Vorrichtung transportiert wird.

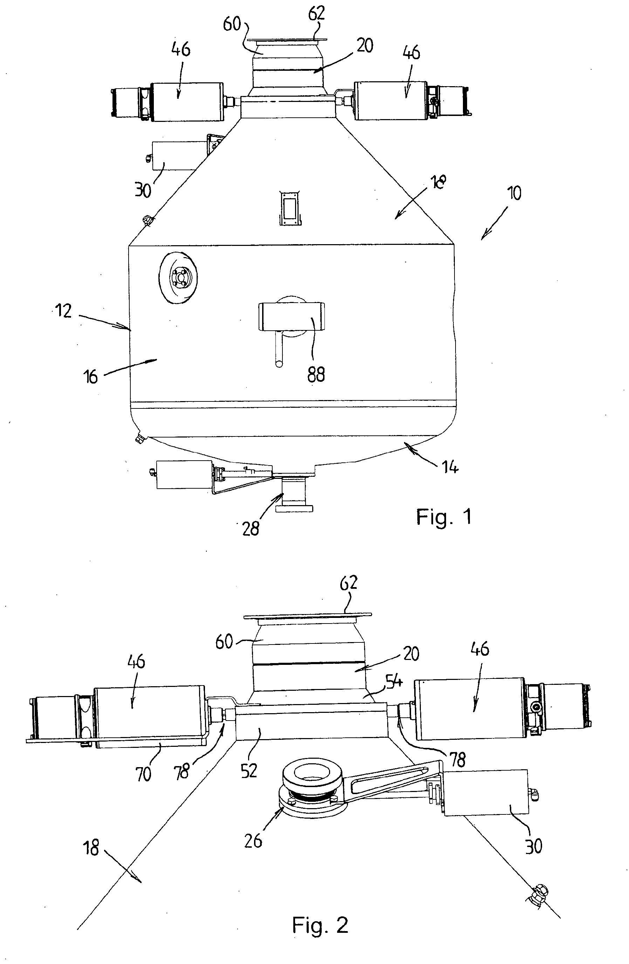

Wie am besten in Fig. 1 dargestellt, weist der druckfeste doppelwandige Behandlungs- und Transportbehälter 10 einen erweiterten Körper 12 auf, der im Wesentlichen aus einem in der aufrechten Stellung des Behälters 10 in Fig. 1 konvex nach unten gewölbten Bodenteil 14, einem zylindrischen Mittelteil 16 und einem nach oben zu verjüngten Oberteil 18 besteht, an dem ein verengter Halsteil 20 angebracht ist.

Der Bodenteil 14, der Mittelteil 16 und der Oberteil 18 sind miteinander verschweißt und umschließen einen Behälterinnenraum 22 flüssigkeitsdicht. Der Halsteil 20 umgibt eine Befüll- und Entleeröffnung 24 mit einem kreisförmigen Öffnungsquerschnitt, durch die der Behälter 10 in einer aufrechten Befüll-Stellung, in der die Befüll- und Entleeröffnung 24 nach oben weist, mit den zu behandelnden Verschlusskappen befüllt wird, und durch die der Behälter 10 nach der Behandlung der Verschlusskappen wieder entleert wird. Die Entleerung erfolgt bei umgedrehtem Behälter 10 in einer Entleer-Stellung, in der die Öffnung 24 nach unten weist. Bei der Entleerung fallen die Verschlusskappen aufgrund ihrer Schwerkraft durch die Öffnung 24 aus dem Behälter 10 heraus.

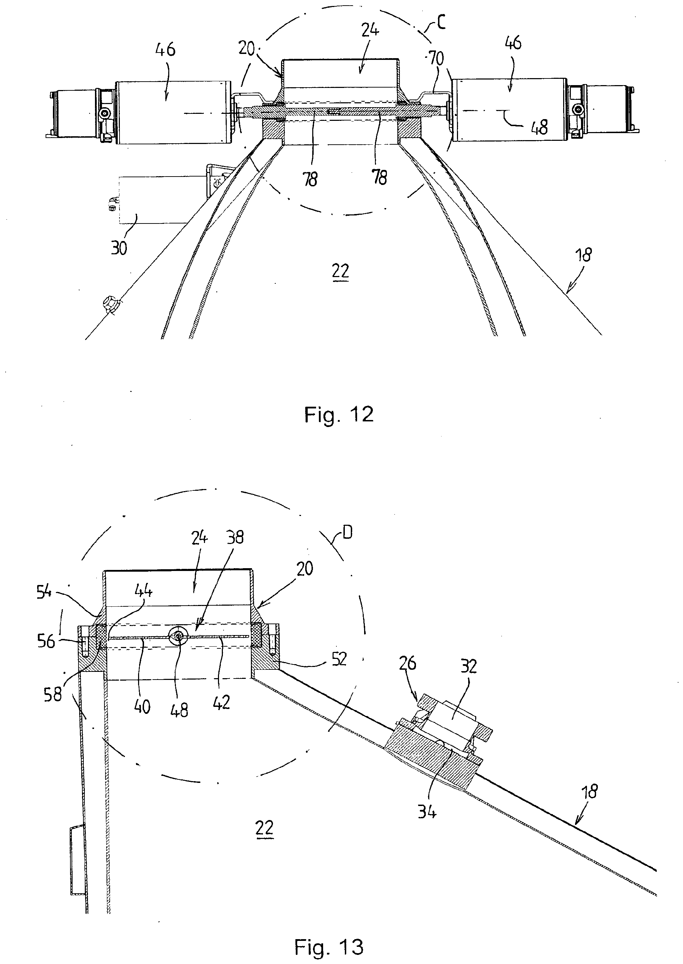

Am Oberteil 18 und am tiefsten Punkt des Unterteils 14 ist jeweils ein Anschlussstutzen 26 bzw. 28 angebracht. Nach der Aufnahme des Behälters 10 in der Reinigungs- und Sterilisationsvorrichtung kann an jedem dieser zwei Stutzen 26, 28 eine Rohrleitung (nicht dargestellt) angeflanscht werden. Durch die Rohrleitungen können Luft, Dampf, Heißwasser, Kaltwasser, ein Wasser-Luft-Gemisch oder andere flüssige oder gasförmige Behandlungsmedien in den Behälter 10 zugeführt bzw. aus dem Behälter 10 abgeführt oder verdrängt werden, so dass sie bei der Behandlung durch den Behälter 10 hindurch strömen. Die beiden Anschlussstutzen 26, 28 umgeben jeweils eine Medienöffnung 32, in der ein Absperrventil 34 angeordnet ist. Das Absperrventil 34 ist jeweils mittels eines Drehantriebs 30 an der Außenseite des Anschlussstutzens 26, 28 betätigbar, um die Medienöffnung 32 zur Zu- bzw. Abfuhr der Behandlungsmedien freizugeben oder zu verschließen, wie in Fig. 13 beispielhaft dargestellt.

Im Behälterinnenraum 22 ist in einem geringen Abstand über dem Bodenteil 14 ein Siebboden (nicht sichtbar) vorgesehen, der einen Hindurchtritt der Behandlungsmedien gestattet und bei aufrechtem Behälter 10 ein Abtropfen der Verschlusskappen erlaubt.

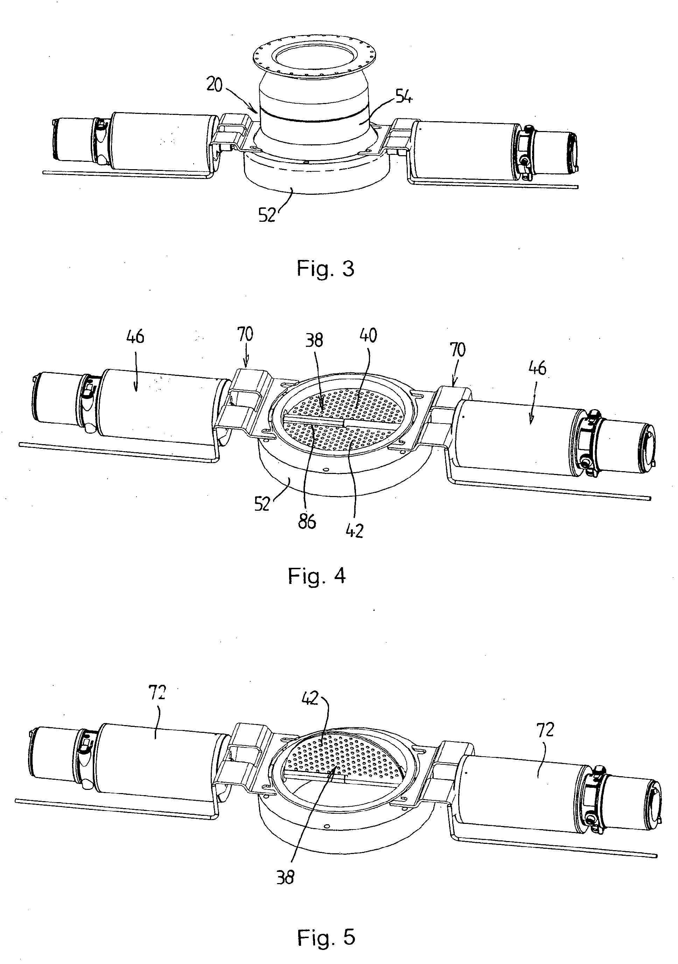

Im Halsteil 20 ist ein Klappenventil 38 mit zwei als Lochblech ausgebildeten, im Umriss halbkreisförmigen Ventilklappen 40, 42 angeordnet, die im geschlossenen Zustand des Ventils 38 (Fig. 4, 7 und 13) in einer Ebene nebeneinander angeordnet sind und die kreisförmige Befüll- und Entleer-Öffnung 24 abgesehen von einem schmalen Spalt 44 entlang der kreisbogenförmigen Ränder der Ventilklappen 40, 42 gemeinsam versperren. Jede der beiden Ventilklappen 40, 42 kann mittels eines an der Außenseite des Halsteils 20 angeordneten steuerbaren Drehantriebs 46 getrennt von der anderen Ventilklappe 42, 40 zwischen zwei Endstellungen um eine gemeinsame Schwenkachse 48 verschwenkt werden. Die Schwenkachse verläuft parallel zu den geraden Rändern der halbkreisförmigen Ventilklappen 40, 42 diametral durch die Befüll- und Entleer-Öffnung 24.

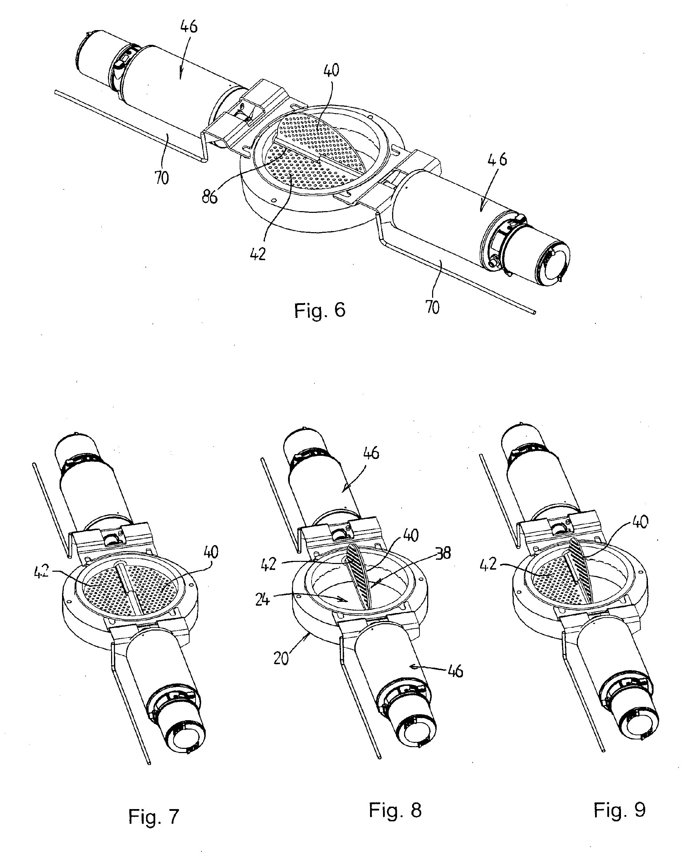

Die beiden Endstellungen können miteinander einen Winkel von 90 Grad einschließen, wie in den Figuren 4 und 5 bzw. 7 und 8 dargestellt. In diesem Fall kann jede der beiden Ventilklappen 40, 42 einzeln oder zusammen mit der anderen Ventilklappe 42, 40 aus der in Fig. 4 und 7 dargestellten ersten Endstellung vom Körper 12 des Behälters 10 weg in die zweite Endstellung verschwenkt werden, in der sie parallel zur Längsmittelachse 50 des Halsteils 20 ausgerichtet ist. In Fig. 6 und 9 befindet sich nur eine der beiden Ventilklappen 40, 42 in der zweiten Endstellung, während sich in Fig. 5 und 8 beide Ventilklappen 40, 42 in der zweiten Endstellung befinden.

In der ersten Endstellung spannen die beiden Ventilklappen 40, 42 eine zur Längsmittelachse 50 des Halsteils 20 senkrechte Ebene auf und versperren die Befüll- und Entleer-Öffnung 24 gemeinsam. In der zweiten Endstellung liegt jede der Ventilklappen 40, 42 in einer zur Längsmittelachse 50 des Halsteils 20 parallelen Ebene, wobei sie auf der vom Körper 12 abgewandten Seite der Schwenkachse 48 angeordnet ist. Wenn sich beide Ventilklappen 40, 42 in der zweiten Endstellung befinden, sind sie parallel zueinander ausgerichtet und liegen mit ihren gegenüberliegenden ebenen Breitseiten gegeneinander an, so dass die Befüll- und Entleer-Öffnung 24 nahezu vollständig freigegeben wird. Wenn sich jeweils eine der beiden Ventilklappen 40, 42 in der ersten bzw. in der zweiten Endstellung befindet, sind die Ventilklappen 40, 42 senkrecht zueinander ausgerichtet und geben die Befüll- und Entleer-Öffnung 24 nur zur Hälfte frei.

Im geschlossenen Zustand des Ventils 38, wie in Fig. 4 und 7 dargestellt, können während der Behandlung Behandlungsmedien durch die Lochbleche der Ventilklappen 40, 42 und den Spalt 44 in den Behälter 10 zugeführt oder aus dem Behälter 10 abgeführt werden. Außerdem können in der Entleer-Stellung des Behälters 10 die behandelten Verschlusskappen im Behälter 10 zurückgehalten werden, während etwaige, im Behälter 10 verbliebene restliche Behandlungsmedien durch die Lochbleche der Ventilklappen 40, 42 und den Spalt 44 aus dem Behälter 10 austreten können.

Im vollständig geöffneten Zustand der Ventilklappen 40, 42, wie in Fig. 5 und 8 dargestellt, können die Verschlusskappen beim Entleeren des Behälters 10 schnell aus diesem abgeführt werden. Wenn die Verschlusskappen beim Entleeren des Behälters 10 langsamer aus diesem ausgetragen werden sollen, ist es möglich, nur eine der Ventilklappen 40, 42 zu öffnen, wie in Fig. 6 und 9 am Beispiel der Ventilklappe 40 dargestellt.

Alternativ können die beiden Endstellungen miteinander auch einen Winkel von 180 Grad einschließen (nicht dargestellt). In diesem Fall sind die beiden Ventilklappen 40, 42 in beiden Endstellungen parallel oder nahezu parallel zueinander und zur Längsmittelachse 50 des Halsteils 20 ausgerichtet, wobei sie in der einen Endstellung auf der vom Körper 12 abgewandten Seite der Schwenkachse 48 und in der anderen Endstellung auf der dem Körper 12 zugewandten Seite der Schwenkachse 48 angeordnet sind. Die beiden Ventilklappen 40, 42 können einzeln oder gemeinsam in einer Mittenstellung zwischen den beiden Endstellungen positioniert werden, in der sie jeweils senkrecht zur Längsmittelachse 50 des Halsteils 20 ausgerichtet sind und die Befüll- und Entleer-Öffnung 24 vollständig oder zur Hälfte versperren, je nachdem ob sich beide Ventilklappen 40, 42 oder nur eine der Ventilklappen 40 oder 42 in der Mittenstellung befinden.

Mit Hilfe der beiden Drehantriebe 46 können die Ventilklappen 40, 42 jedoch auch in beliebige Stellungen zwischen ihren beiden Endstellungen geschwenkt werden, zum Beispiel wenn die Verschlusskappen oder Gegenstände aus dem Behälter 10 langsam in eine Übergabe-Station dosiert werden sollen.

Wie am besten in den Figuren 13 bis 16 dargestellt, besteht der Halsteil 20 aus einem ersten behälterseitigen Abschnitt 52, der einstückig mit dem Oberteil 18 verschweißt ist, einem zweiten stirnseitigen Abschnitt 54, der durch Befestigungsschrauben 56 mit dem ersten Abschnitt 52 verschraubt ist, sowie einem Dichtungsring 58, der zwischen zwei gegenüberliegende schräge Ringschultern der beiden Abschnitte 52, 54 eingesetzt ist, so dass er beim Verschrauben der beiden Abschnitte 52, 54 leicht zusammengepresst wird. Wie in den Figuren 1 bis 3 dargestellt, ist auf den zweiten Abschnitt 54 noch ein kurzer Rohrstutzen 60 mit einem stirnseitigen Schraubflansch 62 aufgesetzt.

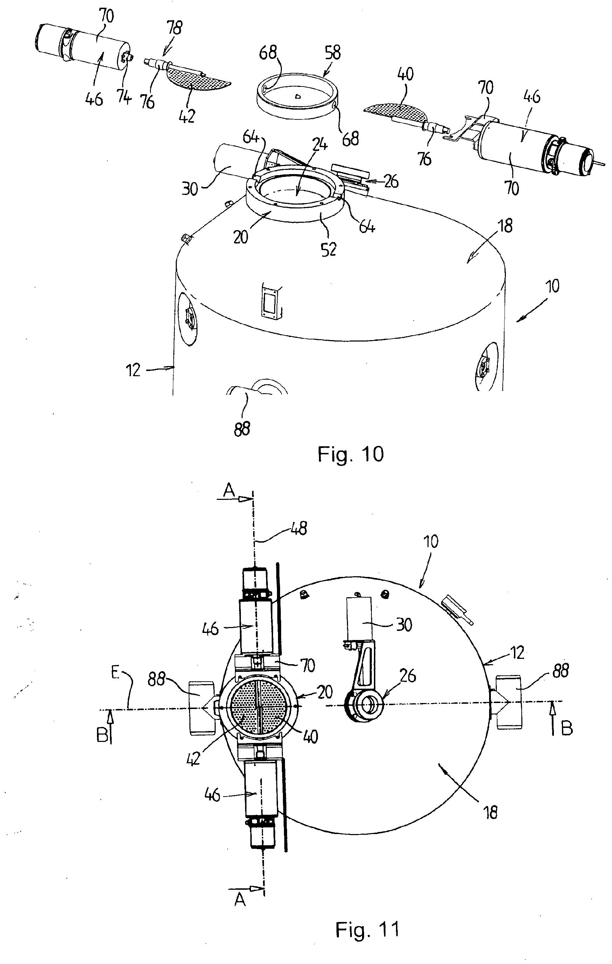

Wie am besten in Fig. 10 am Beispiel des ersten Abschnitts 52 dargestellt, sind die beiden Abschnitte 52, 54 an den einander zugewandten Stirnflächen mit zwei diametral gegenüberliegenden halbkreisförmigen Nuten 64 versehen, die sich nach dem Verschrauben der beiden Abschnitte 52, 54 jeweils zu einer zylindrischen Wandöffnung 66 ergänzen. Der Dichtungsring 58 weist zwei diametral gegenüberliegende radiale Durchgangsöffnungen 68 auf, die nach der Montage mit den Wandöffnungen 66 fluchten.

Wie am besten in den Figuren 1 bis 12 dargestellt, sind die beiden Drehantriebe 46 an diametral entgegengesetzten Seiten des Halsteils 20 angeordnet. Jeder der beiden Drehantriebe 46 ist mittels einer zugehörigen Halterung 70 lösbar am Halsteil 20 befestigt und besteht im Wesentlichen aus einem elektrischen Getriebemotor in einem allgemein zylindrischen Gehäuse 72 sowie einer aus zwei Abschnitten 74, 76 (Fig. 10) bestehenden Antriebswelle 78, die sich vom Getriebemotor durch die benachbarte Wandöffnung 66 des Halsteils 20 und die benachbarte Durchgangsöffnung 68 des Dichtungsrings 58 bis ins Innere des Halsteils 20 erstreckt, wo sie drehfest mit der zugehörigen Ventilklappe 40 bzw. 42 verbunden ist und wo ihr inneres Ende drehbar mit dem inneren Ende der Antriebswelle 78 des anderen Drehantriebs 46 verbunden ist.

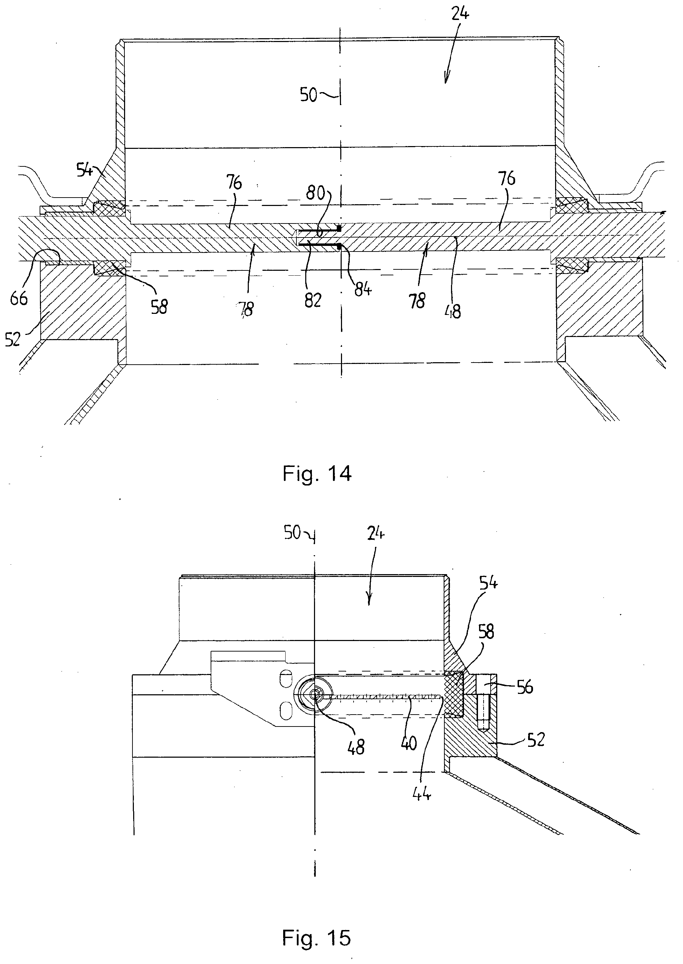

Zum drehbaren Verbinden der beiden Antriebswellen 78 bzw. von deren Abschnitten 76 ist das innere Ende des Abschnitts 76 von einer der Antriebswellen 78 mit einer axialen Zentrier- und/oder Führungsbohrung 80 versehen, in die sich ein überstehender zylindrischer Zentrier- und/oder Führungszapfen 82 am gegenüberliegenden inneren Ende des Abschnitts 76 der anderen Antriebswelle 78 erstreckt, wie am besten in Fig. 14 dargestellt. Zwischen dem Zapfen 82 und der Wand der Bohrung 80 ist eine Passhülse 84 angeordnet.

Wie ebenfalls in Fig. 14 dargestellt, erstreckt sich eine der beiden miteinander sowie mit der Schwenkachse 48 fluchtenden Antriebswellen 78 nach der Montage im Inneren des Halsteils 20 bis zur Längsmittelachse 50, während sich der Zapfen 76 der anderen Antriebswelle 78 über die Längsmittelachse 50 hinaus erstreckt.

Der über den Getriebemotor überstehende Abschnitt 74 und der starr mit der Ventilklappe 40 bzw. 42 verbundene Abschnitt 76 jeder Antriebswelle 78 weisen an ihren gegenüberliegenden freien Enden einen Mehrkantzapfen und eine Mehrkantbuchse auf, in die sich der Mehrkantzapfen einführen lässt, um die beiden Abschnitte 74, 76 drehfest zu verbinden. Die Verbindungsstelle befindet sich außerhalb des Halsteils 20. Auf diese Weise können bei der Montage des Klappenventils 38 zuerst die beiden Ventilklappen 40 und 42 durch Zusammenstecken der daran festgeschweißten Wellenabschnitte 76 verbunden und dann der Dichtungsring 58 montiert werden, wobei die zusammengesteckten Wellenabschnitte 76 unter Verformung des Dichtungsrings 58 von innen her durch dessen Durchgangsöffnungen 68 hindurchgeführt werden. Danach werden der Dichtungsring 58 und die beiden Ventilklappen 40, 42 in den unteren Abschnitt 52 des Halsteils 20 eingesetzt und dann der obere Abschnitt 54 auf dem unteren Abschnitt 52 befestigt. Zuletzt werden die beiden Drehantriebe 46 an der Außenseite des Halsteils 20 montiert und dabei die Mehrkantzapfen und Mehrkantbuchsen der Wellenabschnitte 74 und 76 zusammengesteckt, bevor die Halterungen 70 am Halsteil 20 angebracht werden.

Die durch Stanzen und Umformen aus Blech hergestellten Halterungen 70 weisen jeweils ein dem Halsteil 20 zugewandtes gabelförmiges Ende mit zwei randoffenen Langlöchern für Befestigungsschrauben (nicht dargestellt) auf, mit denen jede Halterung 70 beiderseits von der Wandöffnung 66 für die Antriebswelle 78 des zugehörigen Drehantriebs 46 am Abschnitt 54 festgeschraubt wird, wie am besten in den Figuren 3 bis 10 dargestellt.

Wie am besten in den Figuren 4 bis 6 und 10 dargestellt, erstrecken sich die beiden Antriebswellen 78 bzw. deren Abschnitte 76 innerhalb des Halsteils 20 jeweils entlang von einer Hälfte des geraden Randes der zugehörigen Ventilklappe 40, 42 bis zur Mitte des Randes, wobei sie auf dieser Länge fest mit der Ventilklappe 40 bzw. 42 verschweißt sind. Die andere Hälfte der zugehörigen Ventilklappe 40, 42 steht über das Ende der Antriebswelle 78 bzw. des Abschnitts 76 über und ist nach der Montage des Klappenventils 38 entlang von der anderen Hälfte des geraden Randes durch einen schmalen Spalt 86 vom Abschnitt 76 der anderen Antriebswelle 78 getrennt.

Wie in Fig. 15 dargestellt, liegen diejenigen Breitseiten der beiden Ventilklappen 40, 42, die im geschlossenen Zustand des Klappenventils 38 vom Körper 12 des Behälters 10 abgewandt sind, in einer von der Schwenkachse 48 aufgespannten, zur Längsmittelachse 50 des Halsteils 20 senkrechten Ebene. Daher können die beiden Ventilklappen 40 und 42 in der in Fig. 5 und Fig. 8 dargestellten Endstellung bei vollständig geöffnetem Klappenventil 38 exakt parallel zueinander ausgerichtet werden, wobei sie mit ihren gegenüberliegenden Breitseiten flächig gegeneinander anliegen.

Wenn der Behälter 10 mit dem Halsteil 20 an einen Rapid-Transfer-Port einer Übergabe-Station angedockt werden soll, ist der Halsteil 20 oberhalb vom Klappenventil 38 noch mit einem Verschlussdeckel (nicht dargestellt) versehen, der beim Andocken an den Rapid-Transfer-Port geöffnet wird.

Der Behälter 10 weist am Mittelteil 16 des Körpers 12 zwei diametral entgegengesetzte seitliche Ausleger 88 mit beidseitig offenen zylindrischen Aufnahmebuchsen auf, die bei aufrechtem Behälter 10 parallel und horizontal ausgerichtet sind. Diese Aufnahmebuchsen dienen zur lösbaren Befestigung des Behälters 10 an zwei parallelen Tragdornen, die über eine schwenkbare Halterung der Reinigungs- und Sterilisationsvorrichtung überstehen, so dass der Behälter 10 nach dem Einführen der Tragdorne in die Aufnahmebuchsen um eine zu deren Längsmittelachsen parallele horizontale Schwenkachse schwenkbar ist, wie in der EP 1 449 543 B1 oder der EP 1 510 227 B1 der Anmelderin beschrieben. Die Schwenkbewegung des Behälters 10 erfolgt somit in einer zu den Längsmittelachsen der Tragdorne bzw. Aufnahmebuchsen senkrechten Schwenkebene E (Fig. 11), zu der die Schwenkachse 48 der beiden Ventilklappen 40, 42 wiederum senkrecht ist. Wenn die Befüll- und Entleer-Öffnung 24 zum Entleeren des Behälters 10 schräg nach unten weist, wird so sichergestellt, dass ungeachtet von der Neigungsrichtung des Behälters 10 stets eine der beiden Ventilklappen 40, 42 in der unteren Hälfte der Befüll- und Entleer-Öffnung 24 angeordnet ist. Dadurch wird beim Öffnen des Klappenventils 38 vermieden, dass ein oder mehrere Verschlusskappen oder andere Gegenstände hinter den Abschnitten 76 der Antriebswellen 78 festgehalten werden und im Behälter 10 zurückbleiben.

Bevor das Klappenventil 38 in der Entleer-Stellung des Behälters 10 geöffnet wird, liegen die im Behälter 10 behandelten oder transportierten Verschlusskappen als Schüttung auf den nach oben weisenden Breitseiten der beiden Ventilklappen 40, 42 auf. Beim Öffnen des Ventils 38 werden eine oder beide Ventilklappen 40 und 42 nach unten geschwenkt, d.h. weg von der Schüttung der Verschlusskappen, wodurch diese keinen Druckkräften ausgesetzt wird. Beim Verschwenken der Ventilklappen 40 und 42 fallen die Verschlusskappen durch die Befüll- und Entleer-Öffnung 24 nach unten aus dem Behälter 10, ohne dass die Gefahr einer Verformung von Verschlusskappen in der Schüttung oder einer Brückenbildung besteht. Wenn beide Ventilklappen 40 und 42 synchron geöffnet werden, fallen die Verschlusskappen beiderseits von der Schwenkachse 48 zugleich aus dem Behälter 10, so dass die Entleerung beschleunigt wird.

## Patentansprüche

1. Behandlungs- und Transportbehälter (10) zur Behandlung und zum Transport von kleineren Gegenständen, mit einem erweiterten Körperteil (12) und einem verengten Halsteil (20), der eine Befüll- und Entleer-Öffnung (24) umgibt, sowie mit einem im Halsteil (20) angeordneten Klappenventil (38), das zwei einzeln gesteuert bewegliche Ventilklappen (40, 42) umfasst, die bei geschlossenem Ventil (38) jeweils einen Teil eines Öffnungsquerschnitts der Befüll- und Entleer-Öffnung (24) versperren und zum Entleeren des Behälters (10) unter Freigabe von mindestens einem Teil der Befüll- und Entleer-Öffnung (24) einzeln oder gemeinsam vom Körperteil (12) weg verschwenkbar sind, **dadurch gekennzeichnet, dass** die beiden Ventilklappen (40, 42) fluchtende Schwenkachsen (48) aufweisen, und die beiden Ventilklappen (40, 42) zum Befüllen des Behälters (10) unter Freigabe von mindestens einem Teil der Befüll- und Entleer-Öffnung (24) getrennt oder gemeinsam in Richtung des Körperteils (12) verschwenkbar ausgeführt sind.

2. Behandlungs- oder Transportbehälter nach Anspruch 1 , **dadurch gekennzeichnet, dass** die beiden Ventilklappen (40, 42) dieselbe Größe besitzen und unter gegenseitiger Annäherung bis in eine Endstellung verschwenkbar sind, in der sie parallel zueinander ausgerichtet sind, gegeneinander anliegen und jeweils nahezu eine Hälfte des Öffnungsquerschnitts der Befüll- und Entleer-Öffnung (24) freigeben.

3. Behandlungs- oder Transportbehälter (10) nach einem der vorangehenden Ansprüche, **dadurch gekennzeichnet, dass** die beiden Ventilklappen (40, 42) als Lochbleche ausgebildet sind.

4. Behandlungs- oder Transportbehälter (10) nach einem der vorangehenden Ansprüche, **gekennzeichnet durch** zwei an der Außenseite des Halsteils (20) angeordnete Drehantriebe (46), von denen jeder zum Verschwenken von einer der beiden Ventilklappen (40, 42) dient.

5. Behandlungs- oder Transportbehälter (10) nach Anspruch 4, **dadurch gekennzeichnet, dass** jeder der beiden Drehantriebe (46) eine Antriebswelle (78) aufweist, die sich durch eine Wandöffnung (66) im Halsteil (20) erstreckt und drehfest mit der zugehörigen Ventilklappe (40 bzw. 42) verbunden ist.

6. Behandlungs- oder Transportbehälter (10) nach Anspruch 5, **gekennzeichnet durch** einen in den Halsteil (20) eingesetzten Dichtungsring (58), der zwei diametral gegenüberliegende Durchgangsöffnungen (68) für die beiden Antriebswellen (78) aufweist.

7. Behandlungs- oder Transportbehälter (10) nach Anspruch 5 oder 6, **dadurch gekennzeichnet, dass** die inneren Enden der beiden Antriebswellen (78) drehbar miteinander verbunden sind.

8. Behandlungs- oder Transportbehälter (10) nach einem der Ansprüche 5 bis 7, **dadurch gekennzeichnet, dass** eine der beiden Antriebswellen (78) an ihrem radial inneren Stirnende mit einem Zentrier- oder Führungszapfen (82) versehen ist und dass die andere der beiden Antriebswellen (78) an ihrem radial inneren Stirnende mit einer Zentrier- oder Führungsbohrung (80) für den Führungszapfen (82) versehen ist.

9. Behandlungs- oder Transportbehälter nach einem der Ansprüche 5 bis 8, **dadurch gekennzeichnet, dass** die Befüll- und Entleer-Öffnung (24) einen kreisrunden Öffnungsquerschnitt aufweist, dass jede Ventilklappe (40, 42) einen halbkreisförmigen Umriss mit einem kreisbogenförmigen Rand und einem geraden Rand aufweist, und dass jede Ventilklappe (40, 42) entlang von einer Hälfte ihres geraden Randes mit der zugehörigen Antriebswelle (78) verbunden ist, während der Rest der Ventilklappe (40, 42) über das Ende der zugehörigen Antriebswelle (78) übersteht.

10. Verfahren zum Entleeren eines Transport- oder Behandlungsbehälters (10) nach einem der vorangehenden Ansprüche, wobei der Behälter (10) in eine Entleer-Stellung geschwenkt wird, in welcher der Halsteil (20) nach unten weist, und wobei in der Entleer-Stellung das Klappenventil (38) geöffnet wird, **dadurch gekennzeichnet, dass** beim Öffnen des Klappenventils (38) mindestens eine der Ventilklappen (40, 42) unter Freigabe von mindestens einem Teil der Befüll- und Entleer-Öffnung (24) um fluchtende Schwenkachsen (48) der beiden Ventilklappen (40, 42) vom Körperteil (12) weg nach unten geschwenkt wird.

11. Verfahren nach Anspruch 10, **dadurch gekennzeichnet, dass** beide Ventilklappen (40, 42) mit entgegengesetzter Drehrichtung synchron nach unten geschwenkt werden.

12. Verfahren nach Anspruch 10 oder 11, **dadurch gekennzeichnet, dass** der Behälter (10) in einer Schwenkebene (E) in die Entleer-Stellung geschwenkt wird, die senkrecht zur Schwenkachse (48) der beiden Ventilklappen (40, 42) ist.

## Claims

1. Treatment and transport container (10) for the treatment and transport of small items, with a widened body part (12) and a narrowed neck part (20) which surrounds a filling and emptying opening (24), and with a flap valve (38) arranged in the neck part (20) and comprising two valve flaps (40, 42) which are movable under individual control and which, with the valve (38) closed, each block a part of an opening cross section of the filling and emptying opening (24) and, for the purpose of emptying the container (10), are individually or jointly pivotable away from the body part (12) in order to free at least a part of the filling and emptying opening (24), **characterized in that** the two valve flaps (40, 42) have mutually aligned pivot axes (48), and, for the purpose of filling the container (10), the two valve flaps (40, 42) are designed so as to be individually or jointly pivotable in the direction of the body part (12) in order to free at least a part of the filling and emptying opening (24).

2. Treatment or transport container according to Claim 1, **characterized in that** the two valve flaps (40, 42) have the same size and, on approaching each other, are pivotable as far as end position in which they are oriented parallel to each other, bear against each other and each free almost a half of the opening cross section of the filling and emptying opening (24).

3. Treatment or transport container (10) according to one of the preceding claims, **characterized in that** the two valve flaps (40, 42) are configured as perforated plates.

4. Treatment or transport container (10) according to one of the preceding claims, **characterized by** two rotary drives (46) which are arranged on the outside of the neck part (20) and each of which serves to pivot one of the two valve flaps (40, 42) .

5. Treatment or transport container (10) according to Claim 4, **characterized in that** each of the two rotary drives (46) has a drive shaft (78) which extends through a wall opening (66) in the neck part (20) and which is connected to the associated valve flap (40, 42) for conjoint rotation with the latter.

6. Treatment or transport container (10) according to Claim 5, **characterized by** a sealing ring (58) which is inserted into the neck part (20) and which has two diametrically opposite through-openings (68) for the two drive shafts (78).

7. Treatment or transport container (10) according to Claim 5 or 6, **characterized in that** the inner ends of the two drive shafts (78) are connected rotatably to each other.

8. Treatment or transport container (10) according to one of Claims 5 to 7, **characterized in that** one of the two drive shafts (78) is provided with a centring or guiding pin (82) at its radially inner end, and **in that** the other of the two drive shafts (78) is provided, at its radially inner end, with a centring or guiding bore (80) for the guide pin (82) .

9. Treatment or transport container according to one of Claims 5 to 8, **characterized in that** the filling and emptying opening (24) has a circular opening cross section, **in that** each valve flap (40, 42) has a semi-circular contour with a circular-arc-shaped edge and a straight edge, and **in that** each valve flap (40, 42) is connected, along half of its straight edge, to the associated drive shaft (78), while the rest of the valve flap (40, 42) protrudes past the end of the associated drive shaft (78).

10. Method for emptying a transport or treatment container (10) according to one of the preceding claims, wherein the container (10) is pivoted into an emptying position in which the neck part (20) points downwards, and wherein the flap valve (38) is opened in the emptying position, **characterized in that**, when opening the flap valve (38), at least one of the valve flaps (40, 42) is pivoted downwards away from the body part (12), about aligned pivot axes (48) of the two valve flaps (40, 42), in order to free at least a part of the filling and emptying opening (24).

11. Method according to Claim 10, **characterized in that** both valve flaps (40, 42) are pivoted downwards synchronously in mutually opposite rotation directions.

12. Method according to Claim 10 or 11, **characterized in that** the container (10) is pivoted in a pivot plane (E) into the emptying position, which is perpendicular to the pivot axis (48) of the two valve flaps (40, 42).

## Revendications

1. Récipient de traitement et de transport (10) pour le traitement et pour le transport de tout petits objets, avec une partie de corps élargie (12) et une partie de col rétrécie (20), qui entoure une ouverture de remplissage et de vidange (24), ainsi qu'avec une soupape à clapet (38) disposée dans la partie de col (20), qui comprend deux clapets de soupape mobiles commandés individuellement (40, 42), qui lorsque la soupape (38) est fermée ferment respectivement une partie d'une section transversale d'ouverture de l'ouverture de remplissage et de vidange (24) et qui peuvent être écartés de la partie de corps (12) par pivotement individuellement ou en même temps pour vider le récipient (10) en libérant au moins une partie de l'ouverture de remplissage et de vidange (24), **caractérisé en ce que** les deux clapets de soupape (40, 42) présentent des axes de pivotement alignés (48), et les deux clapets de soupape (40, 42) sont réalisés de façon pivotante séparément ou ensemble en direction de la partie de corps (12) pour le remplissage du récipient (10) avec libération d'au moins une partie de l'ouverture de remplissage et de vidange (24).

2. Récipient de traitement ou de transport selon la revendication 1, **caractérisé en ce que** les deux clapets de soupape (40, 42) présentent la même grandeur et peuvent pivoter avec rapprochement mutuel jusque dans une position finale, dans laquelle ils sont orientés parallèlement l'un à l'autre, s'appliquent l'un contre l'autre et libèrent respectivement pratiquement une moitié de la section transversale d'ouverture de l'ouverture de remplissage et de vidange (24).

3. Récipient de traitement ou de transport (10) selon l'une quelconque des revendications précédentes, **caractérisé en ce que** les deux clapets de soupape (40, 42) sont formés par des tôles perforées.

4. Récipient de traitement ou de transport (10) selon l'une quelconque des revendications précédentes, **caractérisé par** deux entraînements rotatifs (46) disposés sur le côté extérieur de la partie de col (20), parmi lesquels chacun sert pour le pivotement d'un des deux clapets de soupape (40, 42).

5. Récipient de traitement ou de transport (10) selon la revendication 4, **caractérisé en ce que** chacun des deux entraînements rotatifs (46) présente un arbre d'entraînement (78), qui s'étend à travers une ouverture de paroi (66) dans la partie de col (20) et est assemblé sans rotation au clapet de soupape associé (40 ou 42).

6. Récipient de traitement ou de transport (10) selon la revendication 5, **caractérisé par** un anneau d'étanchéité (58) inséré dans la partie de col (20), qui présente deux ouvertures de passage diamétralement opposées (68) pour les deux arbres d'entraînement (78).

7. Récipient de traitement ou de transport (10) selon la revendication 5 ou 6, **caractérisé en ce que** les extrémités intérieures des deux arbres d'entraînement (78) sont assemblées de façon rotative l'une à l'autre.

8. Récipient de traitement ou de transport (10) selon l'une quelconque des revendications 5 à 7, **caractérisé en ce qu'**un des deux arbres d'entraînement (78) est muni à son extrémité frontale radialement intérieure d'un tourillon de centrage ou de guidage (82) et **en ce que** l'autre des deux arbres d'entraînement (78) est muni à son extrémité frontale radialement intérieure d'un trou de centrage ou de guidage (80) pour le tourillon de guidage (82).

9. Récipient de traitement ou de transport (10) selon l'une quelconque des revendications 5 à 8, **caractérisé en ce que** l'ouverture de remplissage et de vidange (24) présente une section transversale d'ouverture ronde, **en ce que** chaque clapet de soupape (40, 42) présente un contour semi-circulaire avec un bord en forme d'arc de cercle et un bord droit, et **en ce que** chaque clapet de soupape (40, 42) est assemblé le long d'une moitié de son bord droit à l'arbre d'entraînement correspondant (78), tandis que le reste du clapet de soupape (40, 42) dépasse au-delà de l'extrémité de l'arbre d'entraînement correspondant (78).

10. Procédé de vidange d'un récipient de transport ou de traitement (10) selon l'une quelconque des revendications précédentes, dans lequel le récipient (10) est basculé dans une position de vidange, dans laquelle la partie de col (20) est orientée vers le bas, et dans lequel dans la position de vidange la soupape à clapet (38) est ouverte, **caractérisé en ce que** lors de l'ouverture de la soupape à clapet (38) au moins un des clapets de soupape (40, 42) est basculé vers le bas autour d'axes de pivotement alignés (48) des deux clapets de soupape (40, 42) en s'écartant de la partie de corps (12) avec libération d'au moins une partie de l'ouverture de remplissage et de vidange (24) .

11. Procédé selon la revendication 10, **caractérisé en ce que** les deux clapets de soupape (40, 42) sont basculés vers le bas de façon synchrone avec un sens de rotation opposé.

12. Procédé selon la revendication 10 ou 11, **caractérisé en ce que** le récipient (10) est basculé dans la position de vidange dans un plan de pivotement (E), qui est perpendiculaire à l'axe de pivotement (48) des deux clapets de soupape (40, 42).
